Europäisches Patentamt

European Patent Office

Office européen des brevets

(19)

(11) **EP 1 402 837 A1**

(12) **EUROPEAN PATENT APPLICATION**

(43) Date of publication:
**31.03.2004 Bulletin 2004/14**

(51) Int Cl.⁷: **A61B 18/14**

(21) Application number: **03255838.9**

(22) Date of filing: **17.09.2003**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **18.09.2002 US 245928**

(71) Applicant: **ETHICON ENDO-SURGERY, INC.**
**Cincinnati, Ohio 45242 (US)**

(72) Inventor: **Long, Gary L.**
**Gerrards Cross Buckinghamshire SL9 7PZ (GB)**

(74) Representative: **Tunstall, Christopher Stephen**
**Carpmaels & Ransford**
**43 Bloomsbury Square**
**London WC1A 2RA (GB)**

(54) **Endoscopic ablation system with a plurality of electrodes**

(57)     A tissue ablation system is described. The tissue ablation system can include a plurality of electrode pairs and a viewing window between each pair of adjacent electrodes. The electrodes can be energized in a predetermined sequence to ablate tissue around the circumference of a body lumen.

FIG. 2

EP 1 402 837 A1

**Description**

Field of the Invention

The present invention relates, in general, to an endoscopic ablation system and, more particularly, to an endoscopic ablation system including a plurality of electrodes adapted to ablate tissue in the esophagus.

Background of the Invention

Gastro-esophageal reflux disease (GERD), which is associated with severe heartburn, affects a substantial portion of the world population. People who experience heartburn at least once a week are reportedly at an increased risk of developing esophageal cancer in their lifetime. When left untreated, chronic GERD can cause the inner lining of the esophagus to change from squamous mucosa to columnar mucosa, which sometimes includes intestinal metaplasia or Barrett's esophagus. Left untreated, Barrett's esophagus can progress to esophageal cancer, for which a common surgical treatment is esophagectomy (removal of the esophagus.)

Accordingly, scientists and engineers continue to seek improved medical instruments for treating diseased tissue in the esophagus.

Summary of the Invention

In one embodiment, the present invention provides an ablation system for treating tissue of a patient. The ablation system comprises a plurality of electrode pairs. Adjacent electrodes provide an ablation index I = P/d of between about 1 and 200, more particularly between about 15 and 35, where P is the perimeter of an electrode and d is the spacing between adjacent electrodes. The ablation system can include a viewing window disposed between each pair of adjacent electrodes. An electric current supply can be provided for providing current to the electrodes according to a predetermined sequence and/or according to a predetermined duration. Adjacent electrodes can be energized, in turn, to view and ablate tissue around the circumference of a body lumen without the need for excessive rotation and repositioning of the electrodes within the body lumen. In one embodiment, the ablation system comprises a sheath, an ablation cap disposed at the distal end of the sheath, a plurality of electrodes disposed on the ablation cap, with a viewing window disposed between each pair of adjacent electrodes.

The present invention also provides a method of treating tissue within the lumen of a patient, such as within the esophagus. The method can comprise the steps of providing a plurality of electrode pairs, wherein adjacent electrodes have an ablation index between about 1 and 200; positioning the electrode pairs within the lumen; and energizing adjacent electrodes to ablate tissue while viewing tissue between the energized electrodes. In one embodiment the method includes the step of collapsing the lumen around the electrodes, such as by using a vacuum.

Brief Description of the Drawings

The novel features of the invention are set forth with particularity in the appended claims. The invention itself, however, both as to organization and methods of operation, together with further objects and advantages thereof, may best be understood by reference to the following description, taken in conjunction with the accompanying drawings in which:

Figure 1 is an illustration of an endoscopic ablation system according to the present invention mounted on a flexible endoscope.

Figure 2 is an enlarged view of an ablation cap at the distal end of the endoscopic ablation system illustrated in Figure 1.

Figure 3 is a geometric diagram showing the relative size and position of two adjacent electrodes that would be mounted on the ablation cap illustrated in Figure 2.

Figure 4 is a sectional view of the lower esophagus and the upper stomach of a human being.

Figure 5 illustrates the use of the endoscopic ablation system of Figure 1 to treat tissue at the lower esophagus.

Figure 6 is sectional view of the lower esophagus showing tissue that has been treated using the endoscopic ablation system of Figure 1.

Figure 7 illustrates an alternative embodiment of an endoscopic ablation system, which includes a rotation knob 58 and a valve 60 (also referred to as a tapered end cover).

Figure 8 is a sectional view of the distal end of the endoscopic ablation system illustrated in Figure 7.

Figure 9 is a sectional view taken at line 9-9 of the endoscopic ablation system illustrated in Figure 8.

Figure 10 is a sectional view taken at line 10-10 of the endoscopic ablation system illustrated in Figure 8.

Figure 11 is an illustration of a further embodiment of an endoscopic ablation system, which includes an electrode sled 70.

Figure 12 is an enlarged, perspective view of the distal portion of the endoscopic ablation system illustrated in Figure 11, showing electrode sled 70 in an extended position.

Figure 13 is an enlarged, perspective view of the distal portion of the endoscopic ablation system illustrated in Figure 11, showing electrode sled 70 in a retracted position.

Figure 14 is an enlarged, top view of the distal portion of the endoscopic ablation system illustrated in Figure 11, showing electrode sled 70 in the extended position.

Figure 15 is an enlarged, sectional side view of the distal portion of the endoscopic ablation system illustrated in Figure 11, showing electrode sled 70 in the extended position.

Figure 16 is an enlarged, end view of the distal portion of the endoscopic ablation system illustrated in Figure 11.

Figure 17 is an illustration of a further embodiment of an endoscopic ablation system, which includes a tapered end cover 84 and a timer 91.

Figure 18 is a sectional view of the distal portion of the endoscopic ablation system shown in Figure 17, wherein a plurality of electrodes 28 are mounted on the tapered end cover 84 near a distal tip 104.

Figure 19 is a sectional view of the distal portion of the endoscopic ablation system shown in Figure 17, wherein a plurality of electrodes 28 are mounted on a rigid support member 26.

Figure 20 is a sectional view of the distal portion of the endoscopic ablation system shown in Figure 17, wherein a plurality of electrodes 28 are mounted partially on rigid support member 26 and partially on tapered end cover 84.

Figure 21 is a sectional view of the proximal portion of the endoscopic ablation system shown in Figure 17.

Figure 22 is a sectional view of the mouth and throat of a patient during intubation of the endoscopic ablation system shown in Figure 17.

Figure 23 is a sectional view of the distal portion of a further embodiment of an endoscopic ablation system, which includes an open-end piece 114 (also referred to as a tapered end cover).

Figure 24 is a graph showing the relationship of an Ablation Quality to an Ablation Index "I", for the endoscopic ablation system according to the present invention.

Figure 25 is an isometric view of ablation cap 20 with a plurality of electrodes 28, which are electrically connected to a control unit 150 and a RF generator.

Figure 26 is a geometric diagram showing the relative size and position of the plurality of electrodes 28 that would be mounted on ablation cap 20 illustrated in Figure 25.

Figure 27 is a sectional view of the distal portion of an endoscopic ablation system 11 that includes an image sensor 120.

Figure 28 is a side view of the distal portion of endoscopic ablation system 11 shown in Figure 27, with a detachable ablation cap 146 removed from a flexible shaft 138.

Detailed Description of the Invention

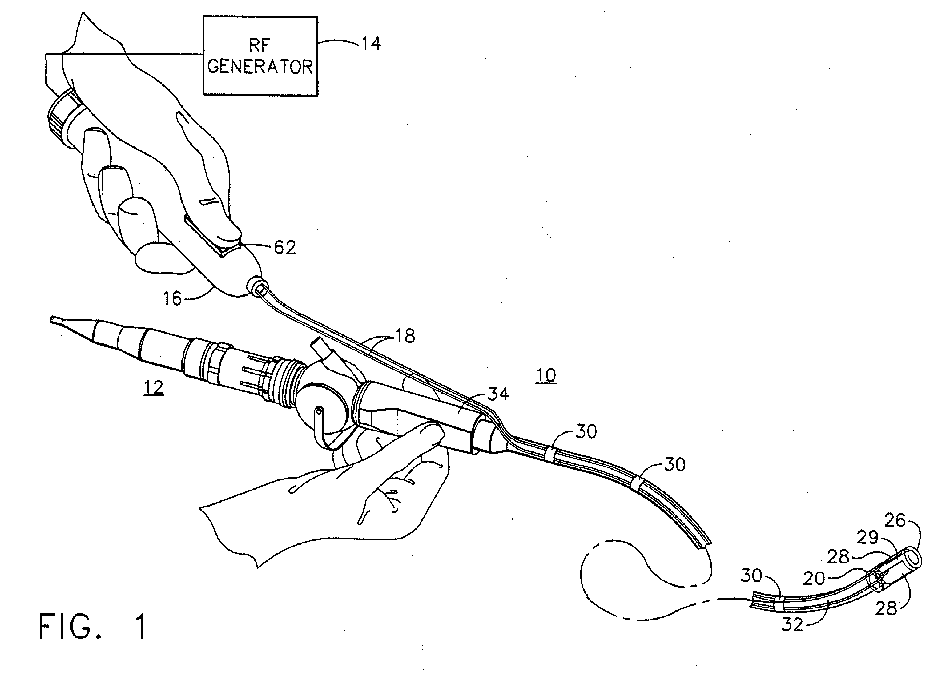

Figure 1 shows an endoscopic ablation system 10 according to the present invention mounted on a flexible endoscope 12 (also referred to as endoscope 12), such as the GIF-100 model available from Olympus Corporation. Flexible endoscope 12 includes an endoscope handle 34 and a flexible shaft 32. Endoscopic ablation system 10 generally comprises an ablation cap 20, a plurality of conductors 18, a handpiece 16 having a switch 62, and an RF (radio frequency) generator 14. Ablation cap 20 fits over the distal end of flexible shaft 32 and conductors 18 attach to flexible shaft 32 using a plurality of clips 30. Ablation cap 20 includes a rigid support member 26, a plurality of electrodes 28, and a viewing window 29 positioned between electrodes 28. In this embodiment, rigid support member 26 is made of a transparent material such as polycarbonate and viewing window 29 is the portion of rigid support member 26 between electrodes 18. Manual operation of switch 62 of handpiece 16 electrically connects or disconnects electrodes 18 to RF generator 14. Alternatively, switch 62 may be mounted on, for example, a foot switch (not shown).

RF generator 14 is a conventional, bipolar/monopolar electrosurgical generator such as one of many models commercially available, including Model Number ICC 350, available from Erbe, GmbH. Either the bipolar mode or the monopolar mode may be used for the present invention. When using the bipolar mode with two electrodes 18 on ablation cap 20, one electrode is electrically connected to one bipolar polarity, and the other electrode is electrically connected to the opposite bipolar polarity. If more than two electrodes 18 are used, polarity of electrodes 18 is alternated so that any two adjacent electrodes have opposite polarities.

When using the monopolar mode with two or more electrodes 18, a grounding pad is not needed on the patient. Because a generator will typical be constructed to operate upon sensing connection of ground pad to the patient when in monopolar mode, it can be useful to provide an impedance circuit to simulate the connection of ground pad to the patient. Accordingly, when the device of the present invention is used in monopolar mode without a grounding pad, an impedance circuit can be assembled by one of skilled in the art, and electrically connected in series with one of conductors 18 that would otherwise be used with a grounding pad during monopolar electrosurgery. Use of the impedance circuit allows use of the generator in monopolar mode without use of a grounding pad attached to the patient.

The optimal power level required to operate endoscopic ablation system 10 of the present invention is approximately in the range of 10-50 watts, although endoscopic ablation system 10 is also functional at lower or higher power levels.

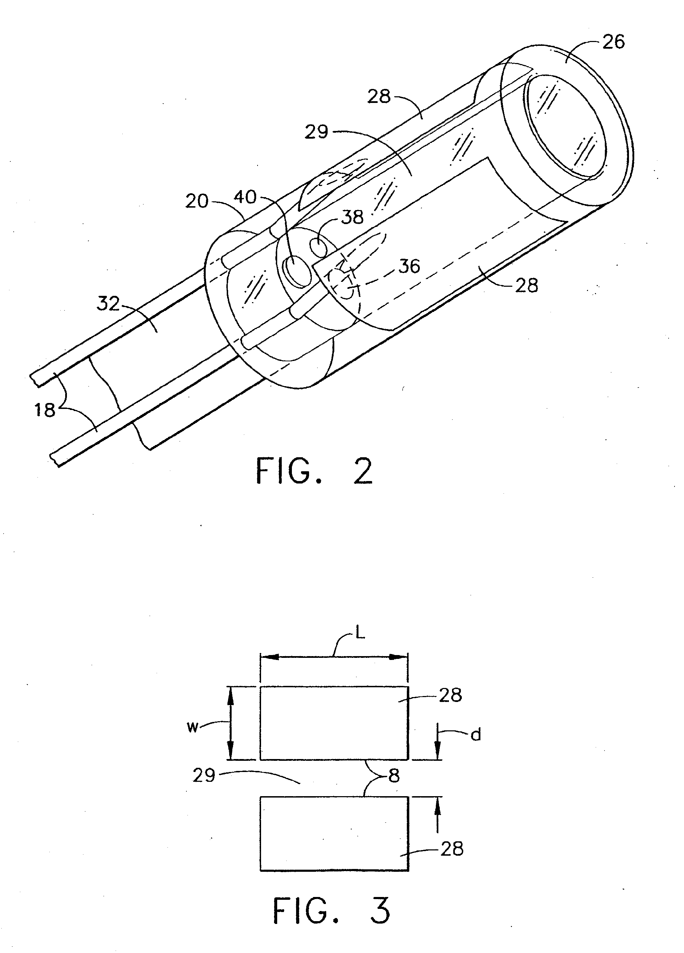

Figure 2 is an enlarged view of ablation cap 20 of endoscopic ablation system 10 shown in Figure 1. Ablation cap 20 fits securely over the distal end of flexible shaft 32. Electrodes 28 are positioned on the outside surface of rigid support member 26, which has a circular cylinder shape in this embodiment. Rigid support member 26 may also have alternate cylindrical shapes, including shapes in which at least a portion of the cross sectional perimeter is non-arcuate. For example, rigid support member 26 may have a "D-shape" cross-section, where electrodes 28 are positioned on the flat portion of the "D-shape." Conductors 18 are electrically insulated from each other and surrounding structures, except for electrical connections such as to electrodes 28. The distal end of flexible shaft 32 of flexible endoscope 12 includes a light source 40, a viewing port 38, and a working channel 36. Viewing port 38 transmits an image within its field of view to an optical device such as a CCD camera within flexible endoscope 12 so that an operator may view the image on a display monitor (not shown). In the embodiment shown in Figure 2, the distal end of flexible shaft 32 is proximal to electrodes 28 and viewing window 29, enabling the operator to see tissue between electrodes 28 through viewing window 29.

Figure 3 shows the geometric relationship of a particular embodiment of electrodes 28. In this embodiment, two rectangular electrodes 28, each having a width "w" and a length "L", have parallel, adjacent edges 8 that are separated by a distance "d". This geometric relationship may be used to calculate an ablation index, which has particular significance to the location, size, shape, and depth of ablation achievable, as will be described later. Viewing window 29 (see Figure 2) is approximately defmed by the d x L rectangular area between electrodes 28.

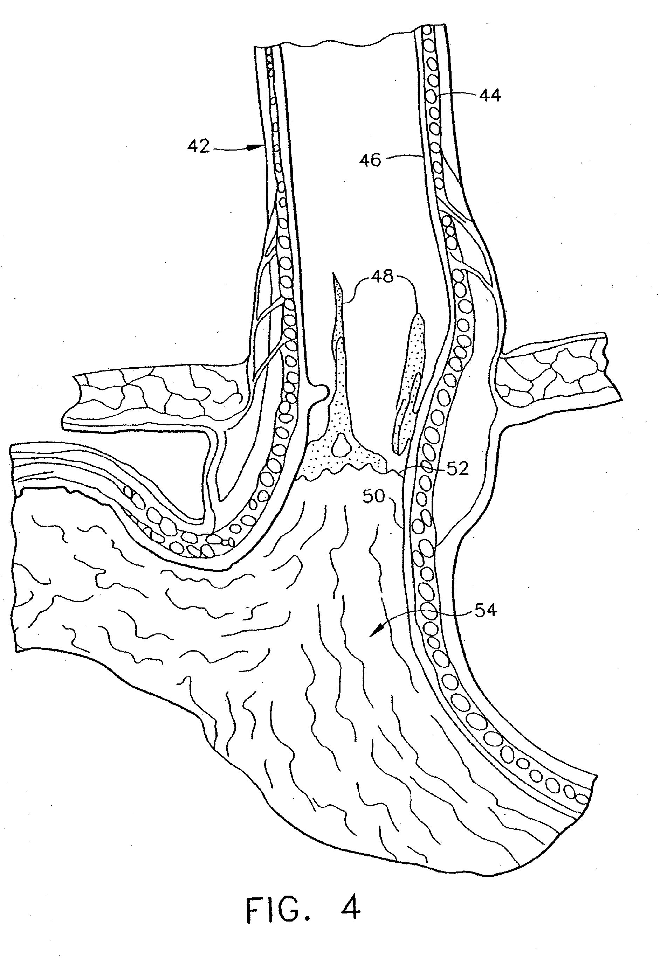

Figure 4 is a sectional view of the lower end of an esophagus 42 and the upper portion of a stomach 54 of a human being. Esophagus 42 has a mucosal layer 46, a muscular layer 44, and a region of diseased tissue 48. The boundary between mucosal layer 46 of esophagus 42 and a gastric mucosa 50 of stomach 54 is a gastro-esophageal junction 52, which is approximately the location for the lower esophageal sphincter (LES). The LES allows food to enter the stomach 54 while preventing the contents of stomach 54 from refluxing into lower esophagus 42 and damaging mucosal layer 46. Diseased tissue 48 can develop when chronic reflux is not treated. In one form, diseased tissue 48 may be, for example, intestinal metaplasia, which is an early stage of Barrett's esophagus. As can be seen in Figure 4, the esophagus is relatively flaccid and contains numerous folds and irregularities on the interior lining.

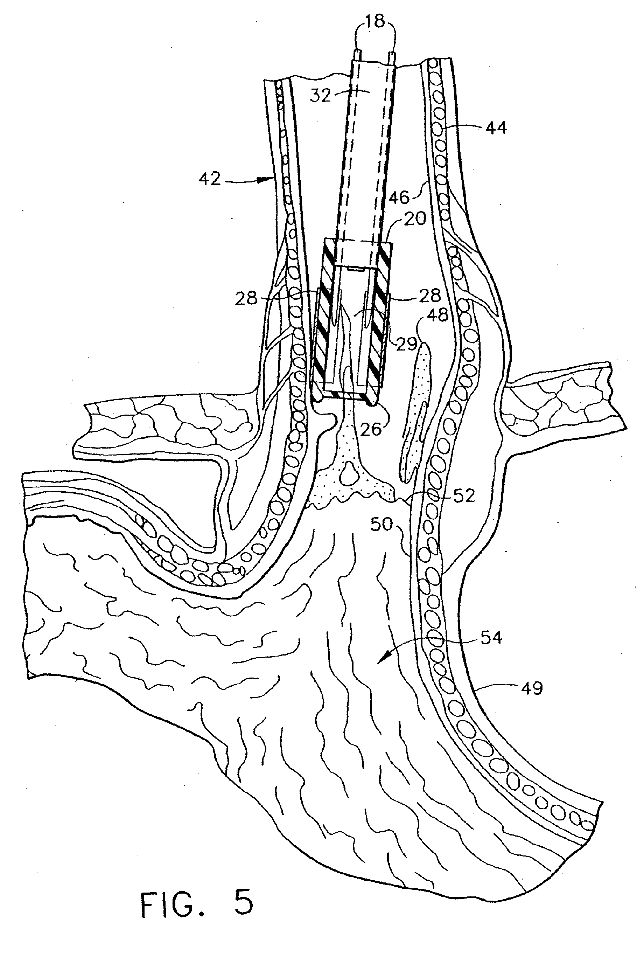

Figure 5 illustrates the use of endoscopic ablation system 10 to treat diseased tissue 48 in lower esophagus 42. The operator positions ablation cap 20 using endoscopic visualization so that diseased tissue 48 to be treated lies under viewing window 29.

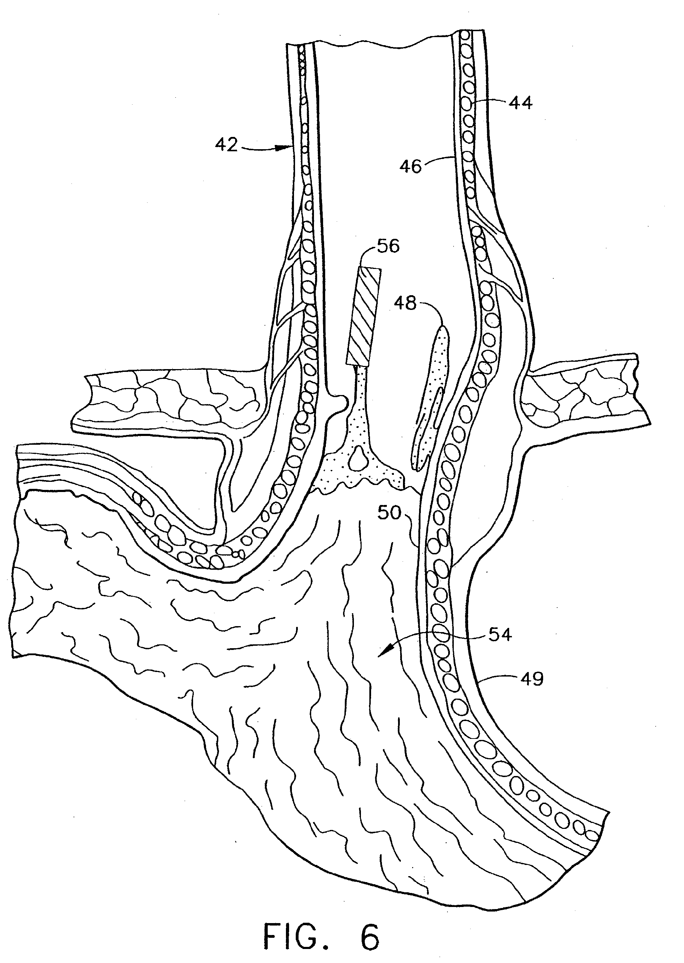

Figure 6 is sectional view of lower esophagus 42 showing tissue that has been treated using endoscopic ablation system 10 according to the present invention. In Figure 6, the size and shape of the treated tissue 56 substantially corresponds to the size and shape of viewing window 29.

The operator may treat diseased tissue 48 using the embodiment of endoscopic ablation system 10 of the present invention shown in Figures 1 and 5 as follows. The operator inserts flexible shaft 32 of endoscope 12 into lower esophagus 42 trans-orally. Rigid support member 26 holds lower esophagus 42 open as the operator uses endoscopic visualization through ablation cap 26 to position electrodes 28 next to the diseased tissue 48 to be treated. Rigid support member 26 opens and supports a portion of the flaccid, lower esophagus 42 and helps to bring the tissue to be treated into intimate contact with electrodes 28 and viewing window 29. While watching through viewing window 29, the operator actuates switch 62, electrically connecting electrodes 28 to RF generator 14 through conductors 18. Electric current then passes through the diseased tissue positioned in viewing window 29. When the operator observes that the tissue in viewing window 29 has been ablated sufficiently, the operator deactuates switch 62 to stop the ablation. The operator may reposition electrodes 28 for subsequent tissue treatment, or may withdraw ablation cap 26 (together with flexible endoscope 12). As illustrated in Figure 6, treated tissue 56 has substantially the same width and length as viewing window 29.

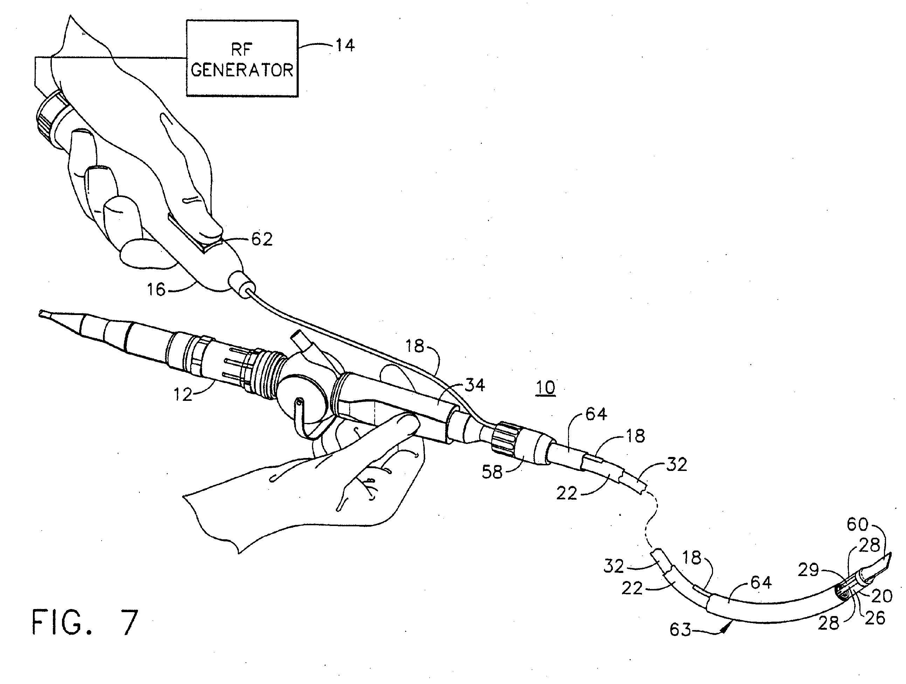

Figure 7 shows an alternate embodiment of an endoscopic ablation system 10 and generally comprises an ablation cap 20, a sheath 63, a pair of conductors 18, a handpiece 16 having a switch 62, and an RF generator 14. An operator may rotate ablation cap 20 around flexible shaft 32 of flexible endoscope 12 by manipulation of a rotation knob 58, which connects to sheath 63. Ablation cap 20 includes a rigid support member 26, at least two electrodes 28, and at least one viewing window 29 (between each pair of adjacent electrodes). Sheath 63 comprises a rotation tube 22 covered by an external tube 64. Ablation cap 20 attaches directly to the distal end of sheath 63. Rotation tube 22 can be made from a material such as, for example, corrugated polyethylene tubing, and fits slidably over a conventional, flexible endoscope. External tube 64 is preferably made from a heat-activated shrink tube material such as polyolefin. Conductors 18 are spirally wrapped around rotation tube 22 prior to assembling and shrinking external tube 64 onto rotation tube 22, thereby tightly retaining conductors 18 in the wound configuration. In the embodiment shown in Figure 7, a valve 60 (also referred to as a tapered end cover), which may be, for example, a duck bill valve, connects to the distal end of rigid support member 26. Valve 60 allows an operator to extend the distal end of flexible endoscope 12 beyond the distal end of rigid support member 26 to improve visualization of tissue structures, especially during intubation. The operator may also retract the distal end of flexible endoscope 12 within rigid support member 26 to allow visualization of viewing window 29 and electrodes 28, while preventing bodily fluids from entering rigid support member 26 and impairing visualization by contact with flexible endoscope 12.

Alternate embodiments of valve 60 may be envisioned by those skilled in the art, each embodiment being particularly adapted to the medical procedure and anatomical structures involved. For example, in an alternative embodiment of the present invention, the distal end of valve 60 could be further tapered and elongated to allow for easier insertion into the esophagus. Valve 60 could further be transparent to enable the physician to visualize through valve 60 during intubation into the esophagus, while preventing contact of bodily fluids against the distal end of flexible endoscope 12.

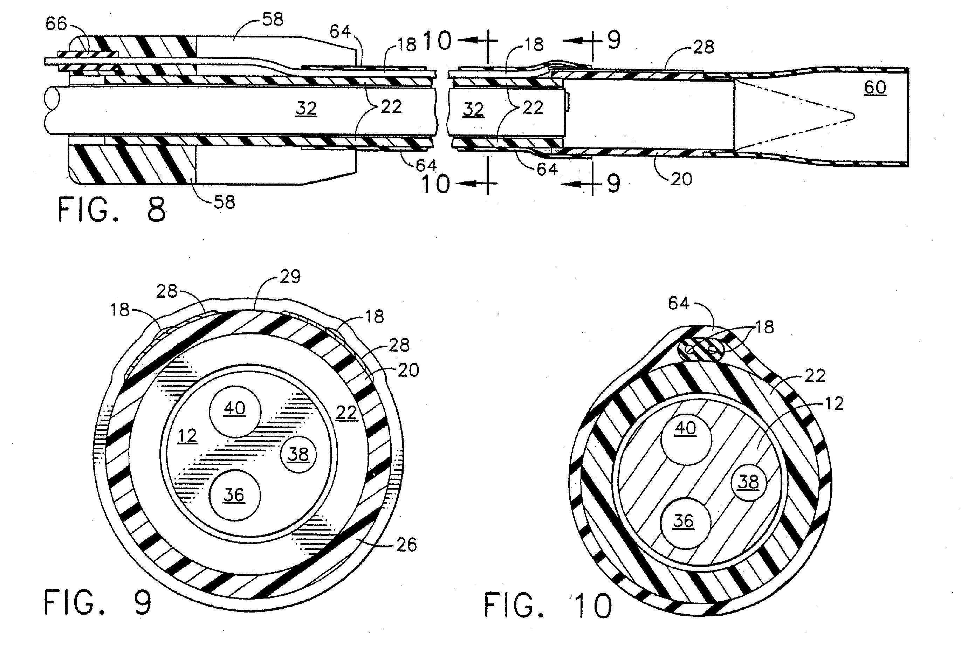

Figure 8 is a sectional view taken along the longitudinal axis of endoscopic ablation system 10 of Figure 7. The distal portion of flexible shaft 32 is inside rotation tube 22 of endoscopic ablation system 10. A pair of conductors 18 passes through a strain relief 66 of rotation knob 58 and between external tube 64 and rotation tube 22. Each conductor 18 connects electrically to one of electrodes 28 on ablation cap 20. Rotation tube 22 rotatably joins rotation knob 58 to ablation cap 20, enabling the operator to rotatably orient electrodes 28, even after insertion into the esophagus, by remotely actuating rotation knob 58. The distal end of flexible shaft 32 extends from the distal end of sheath 63 into ablation cap 20 and proximal to electrodes 18. A viewing window 29 between electrodes 28 is within the field of view of flexible endoscope 12, thus enabling the operator to see on a display monitor the tissue that is located between electrodes 18. Valve 60 extends from the distal end of ablation cap 20 to prevent tissue or fluids from entering ablation cap 20.

Figure 9 is a sectional view taken along line 9-9 of ablation cap 20 of endoscopic ablation system 10 of Figure 8. Conductors 18 connect to electrodes 28 with the portion of rigid support member 26 between electrodes 28 defining viewing window 29. Rotation tube 22 retains flexible shaft 32. The inside diameter of rotation tube 22 is larger than the outer diameter of flexible endoscope 12 to allow rotation of rotation tube 22 while holding flexible endoscope 12 stationary, or vice versa. In this embodiment at least the portion of rigid support member 26 that forms viewing window 29 is transparent so that the operator may endoscopically view the tissue between electrodes 28. Flexible endoscope 12 includes a light source 40, a viewing port 38, and a working channel 36.

Figure 10 is a sectional view taken along line 10-10 of rotation tube 22 of endoscopic ablation system 10 of Figure 8. External tube 64 and rotation tube 22 assemble and retain conductors 18 as already described. Light source 40, viewing port 38, and working channel 36 of flexible endoscope 12 are shown.

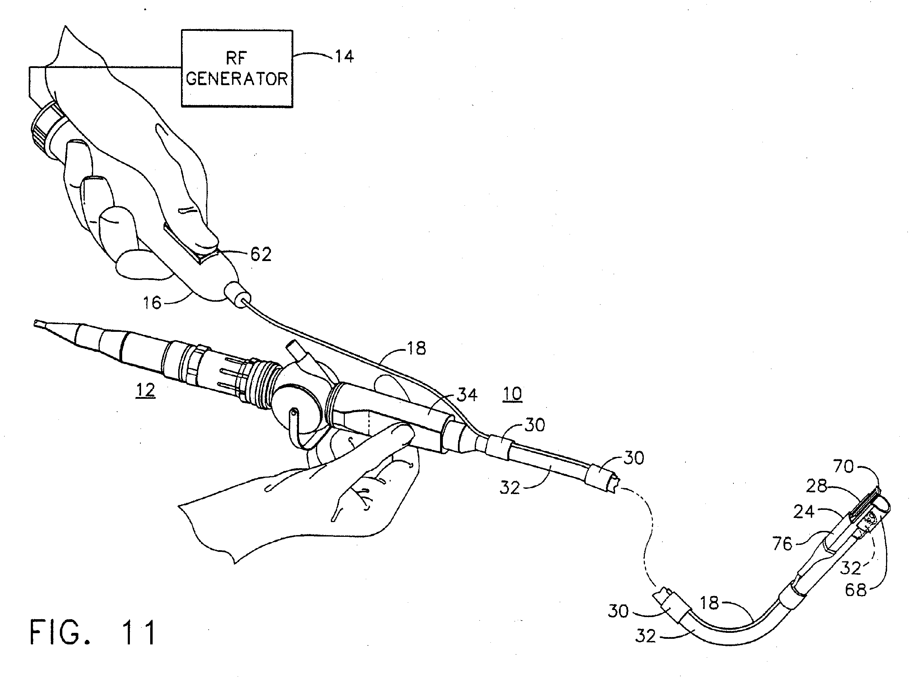

Figure 11 shows a further embodiment of an endoscopic ablation system 10 according to the present invention. A flexible ablation cap 24 includes a flexible support member 68 and at least two electrodes 28 mounted on an electrode sled 70, which may be housed in or extended from a sled housing 76. Flexible ablation cap 24 mounts over the distal end of flexible shaft 32. Conductors 18 electrically connect to electrodes 28 as in the previous embodiments, and may be attached to flexible shaft 32 by a plurality of clips 30. Again, conductors 18 electrically connect to RF generator 14 by a switch 62 of a handpiece 16.

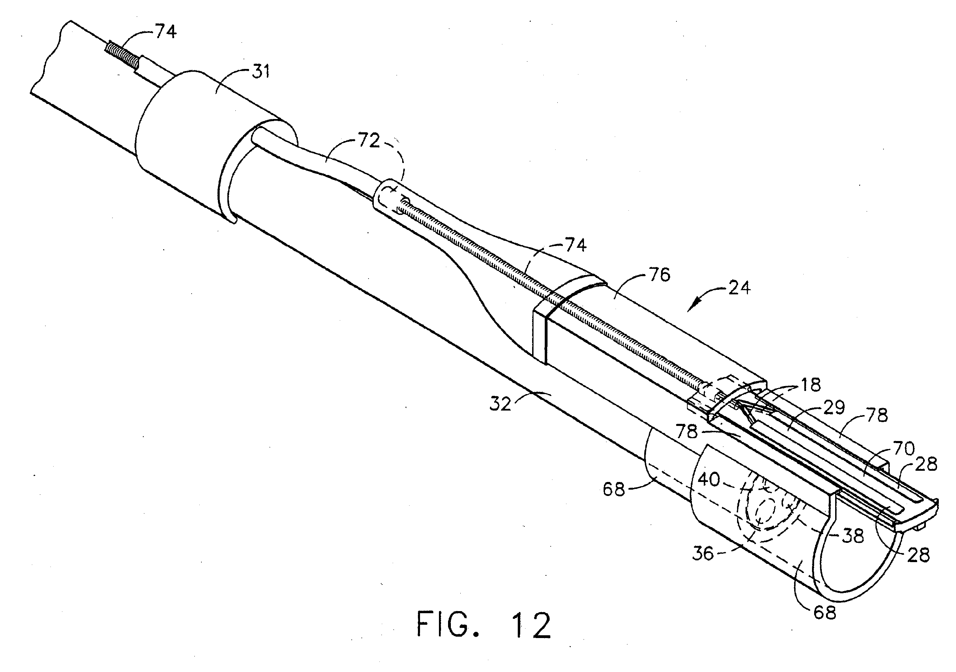

Figure 12 is an enlarged view of flexible ablation cap 24 of the endoscopic ablation system 10 illustrated in Figure 11 with electrode sled 70 fully extended. A sled housing 76 is a soft and flexible, pouch-like container, which may be made of a material such as PTFE in order to prevent damage to the mucosa as the operator introduces endoscopic ablation system 10 into the esophagus. Sled housing 76 and flexible support member 68 may be molded as a single piece. Electrode sled 70 may be made of a clear rigid material such as, for example, polycarbonate. As shown in Figure 12, electrode sled 70 includes two electrodes 28, a viewing window 29, and two conductors 18. At least the portion of electrode sled 70 that forms viewing window 29 is transparent to allow the operator to view endoscopically the tissue between electrodes 28. Flexible support member 68 includes sled guides 78, which are adapted to receive electrode sled 70. Extension of sled 70 to an extended position stiffens flexible support member 68 such as may be desired during ablation; retraction of sled 70 to a retracted position allows flexible support member 68 to flex such as may be desirable during intubation. A drive cable 74 retains conductors 18, which extends proximally through sled housing 76 and into a sleeve 72. Sleeve 72 attaches to flexible shaft 32 by a fixed clip 31. Thus, by extending drive cable 74, electrode sled 70 moves distally and, by retracting drive cable 74, electrode sled 70 moves proximally into sled housing 76.

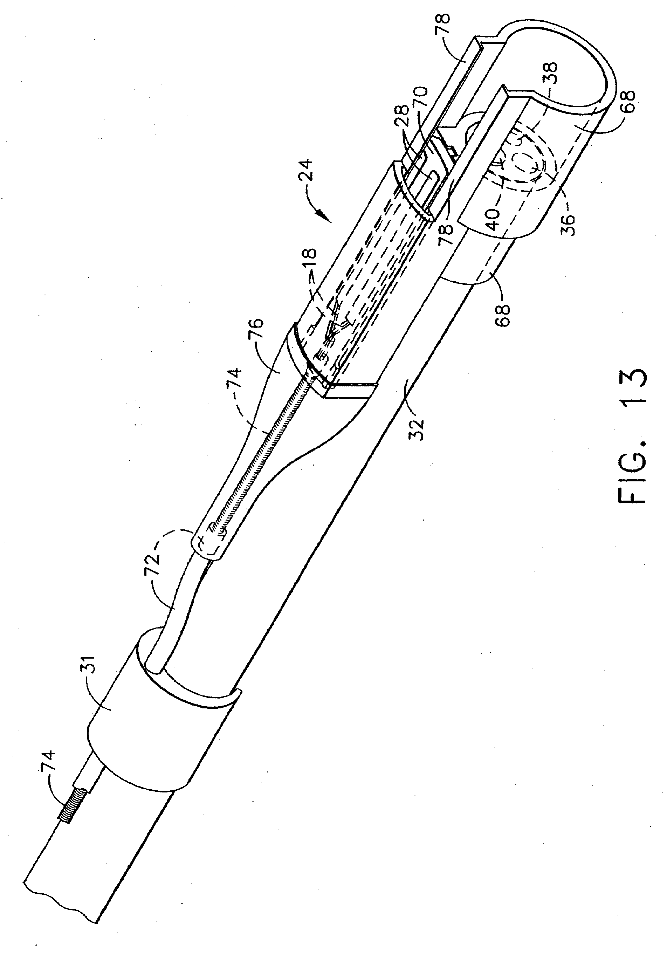

Figure 13 shows flexible ablation cap 24 of endoscopic ablation system 10 of Figure 11 with electrode sled 70 retracted into sled housing 76, or in a retracted position.

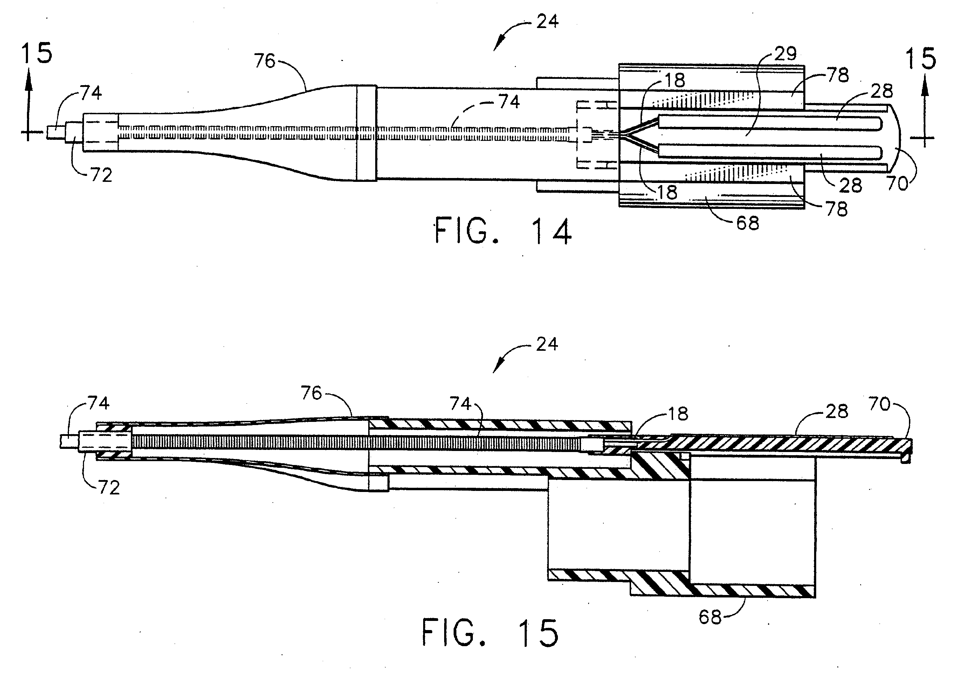

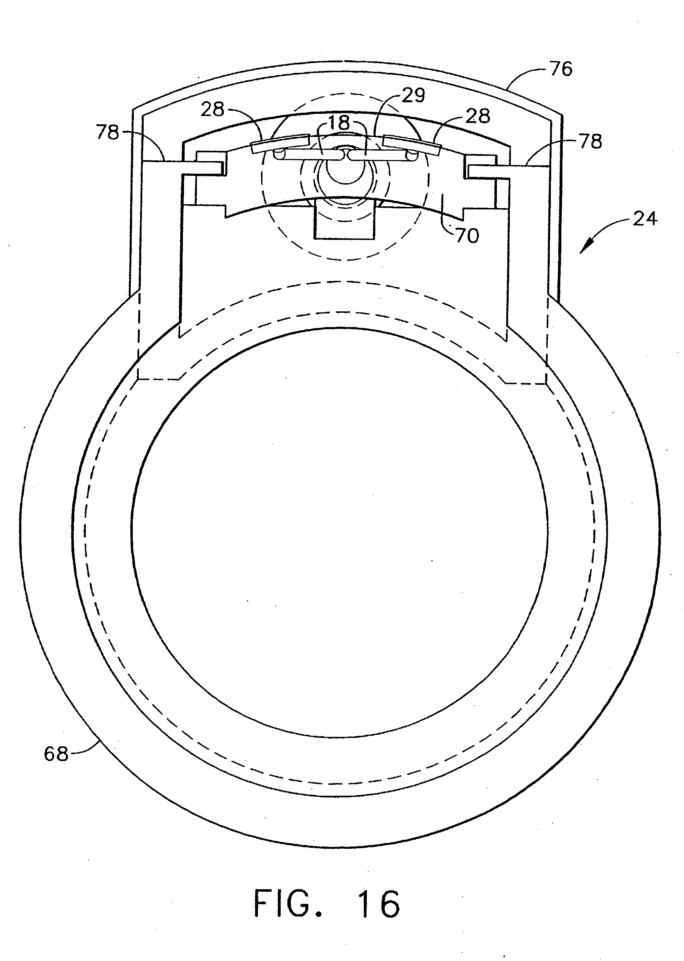

Figures 14-16 are additional views of flexible ablation cap 24 illustrated in Figure 11. Figure 14 is a top view of flexible ablation cap 24 with electrode sled 70 in an extended position. Figure 15 is a side view of flexible ablation cap 24 with electrode sled 70 in an extended position. In Figures 14 and 15 electrode sled 70 includes electrodes 28, viewing window 29 and conductors 18, which are connected to electrodes 28. Flexible support member 68 includes sled guides 78. Drive cable 74, which houses conductors 18, is in turn housed within sled housing 76 and extends proximally into sleeve 72. Figure 16 is an end view of the flexible ablation cap 24 of the endoscopic ablation system 10 illustrated in Figure 11. Figure 16 illustrates the arrangement of sled guides 78 and the engagement of electrode 70 by sled guides 78.

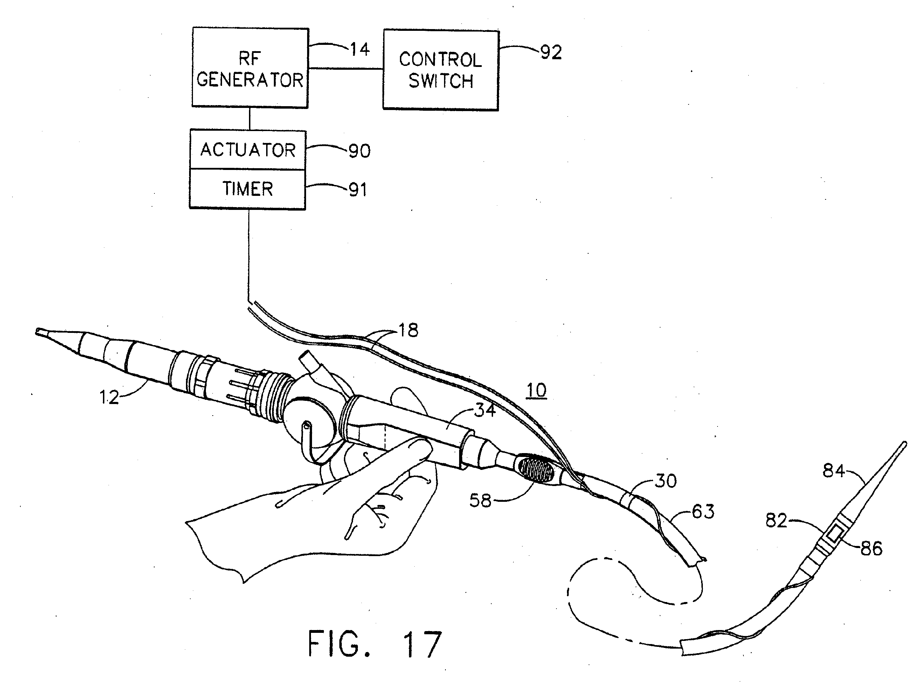

Figure 17 is an illustration of a further embodiment of an endoscopic ablation system 10 for use with an endoscope 12 having an endoscope handle 34. Endoscopic ablation system 10 generally comprises a rotation knob 58, a sheath 63, an ablation cap 82, and a tapered end cover 84. Ablation cap 82 further includes an ablation cap-opening 86. Conductors 18 spirally wrap around the outside of sheath 63 in this embodiment, and at least one clip 30 attaches conductors 18 to sheath 63. Endoscopic ablation system 10 further comprises an actuator 90 and a timer 91. A plurality of electrodes 28 (hidden in this view) on ablation cap 82 electrically connect, via a pair of conductors 18, to actuator 90. The operator actuates actuator 90 manually to enable timer 91 to electrically connect electrodes 28 to RF generator 14 for a predetermined period of time. The operator then actuates control switch 92, which may be a foot operated control switch commonly available with RF generators, to activate RF generator 14. When RF generator 14 is activated, timer 91 automatically connects RF generator 14 to electrodes 28 for a predetermined length of time. For the embodiments of an endoscopic ablation system described herein, an appropriate predetermined length of time is approximately in the range of 0.1 to 10 seconds, and is preferably about one second. However, the length of predetermined time may vary depending on the geometry of the electrodes, the power level used on the RF generator, the type of tissue being treated, and other factors. Timer 91 includes a conventional timer circuit that is connected in electrical series to the output of a RF generator 14 having a control switch 92. When the operator actuates control switch 92, the electrical current from RF generator 14 induces a secondary current inside of timer 91. This secondary current supplies and immediately activates the timer circuit of timer 91, thereby connecting the output of RF generator 14 to electrodes 28 via a relay inside of timer 91. After a predetermined period of time, the relay disengages automatically, therefore electrically disconnecting RF generator 14 from the electrodes 28. Therefore, the operator controls when electrodes 28 are energized to begin ablation of tissue, but timer 91 controls when ablation stops, even though the operator may still be activating control switch 92. Timer 91 ensures complete ablation of diseased tissue in the viewing window and greatly reduces the possibility of operator error associated with RF energy application.

Timer 91 and actuator 90 of Figure 17 may be provided as a handle with a switch much like handle 16 and switch 62 of Figure 1. Alternately, timer 91 and actuator 90 may be incorporated into a table top unit (not shown), combined with RF generator 14 and control switch 92, or electronically packaged in many other ways that are readily apparent to one skilled in the art. Actuator 90, timer 91, RF generator 14, and control switch 92 may comprise a reusable portion of endoscopic ablation system 10. The remaining portion that includes conductors 18, sheath 63, rotation knob 58, and ablation cap 82 may be provided, for example, as a relatively low cost, sterile device that is disposable after use on one patient.

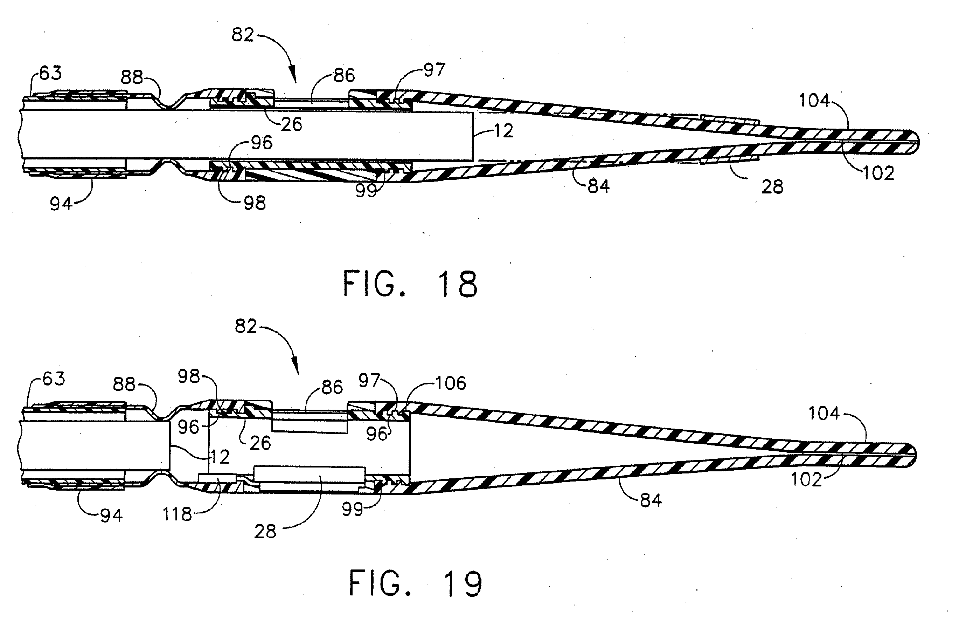

Figures 18, 19, and 20 are sectional views of the distal portion of endoscopic ablation system 10 shown in Figure 17, and illustrate alternate locations of electrodes 28. Figures 18, 19, and 20 show the distal end of sheath 63 inserted into the proximal end of a flexible coupling 88 and attached by a ring 94 tightly compressed around sheath 63 and the proximal end of flexible coupling 88. The distal end of flexible coupling 88 attaches to the proximal end of a rigid support member 26 of ablation cap 82 by the engagement of a plurality of annular projections 96 on the inside of the distal end of flexible coupling 88 with a like plurality of annular grooves 98 formed into the proximal end of rigid support member 26. Flexible coupling 88 is made of a flexible tube material such as silicone rubber and allows low force angulation of sheath 63 with respect to ablation cap 82, thus facilitating passage of ablation cap 82 through the esophagus of the patient. The distal end of rigid support member 26 includes a plurality of annular grooves 99 for retaining a plurality of annular projections 97 on the inside of the proximal end of tapered end cover 84. Tapered end cover 84 is made of a transparent, flexible material such as, for example, clear or tinted polyurethane that is commonly used for flexible, extruded tubing. Tapered end cover 84 further includes an elongated, distal tip 104 that helps the operator to insert ablation cap 82 into the esophagus.

Tapered end cover 84 is hollow in order to allow positioning of the distal end of endoscope 12 partially into tapered end cover 84, as shown in Figure 18. This enables the operator to view the interior of the esophagus, yet protects the distal end of endoscope 12 from tissue structures and bodily fluids that may impair visualization. Tapered end cover 84 is shaped like a bougie tube, which is commonly used by endoscopists for dilating the esophagus prior to intubation with an endoscope. Distal tip 104 of tapered end cover 84 includes a channel 102 so that the operator may pass a guide wire through ablation cap 82 and sheath 63, in order to facilitate positioning of ablation cap 82 inside of the esophagus.

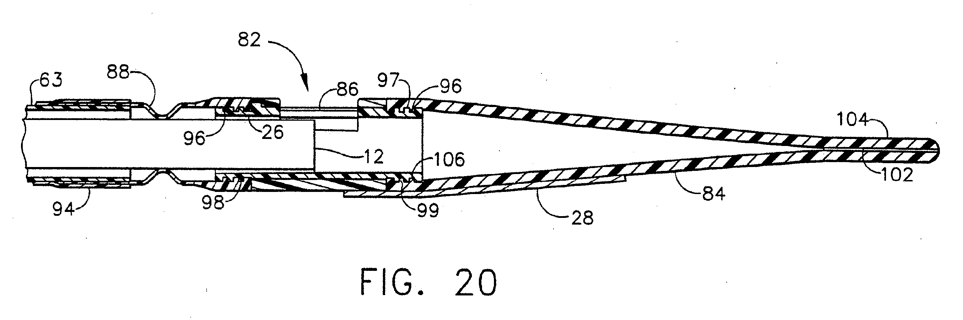

As shown in Figures 18, 19, and 20, electrodes 28 may be mounted at varying locations on ablation cap 82. In Figure 18, electrodes 28 are attached to the outside of tapered end cover 84 near distal tip 104. As indicated in Figure 18, electrodes 28 are positioned on a portion of tapered end cover 84 that has a smaller cross-sectional diameter than the diameter of the distal end of endoscope 12. As shown in Figure 19, electrodes 28 may also be attached to rigid support member 26, as was also described for the embodiments shown in Figures 1 and 7. In Figure 19, a portion of one of conductors 18 is shown as it may be electrically connected to one of electrodes 28 by a solder and/or compression connection. (Conductors 18 are not shown in Figures 18 and 20.) In Figure 20, electrodes 28 are positioned partially on rigid support member 26 and partially on tapered end cover 84. Electrodes 28 may vary in size, shape, and position on ablation cap 82, as shown in the examples of Figures 18, 19, and 20, but importantly, still follow the geometric relationships described for Figure 3 in order to achieve a desired ablation quality.

Still referring to Figures 18, 19, and 20, rigid support member 26 also includes side opening 86. In the examples shown, side opening 86 is rectangularly shaped and positioned between the distal end of flexible coupling 88 and the proximal end of tapered end cover 84. In the examples shown in Figures 19 and 20, side opening 86 is on the side of rigid support member 26 opposing the position of electrodes 26. Side opening 86 provides access to tissue structures next to ablation cap 82 with instrumentation passed through the working channel of endoscope 12. In addition, side opening 86 allows fluid communication between endoscope 12 (that normally includes suction and irrigation channels) and the interior of the esophagus around ablation cap 86. Therefore, the operator may position electrodes 28 adjacent to tissue to be ablated and apply the suction provided with endoscope 12. As the lumen size of the esophagus decreases under vacuum, the esophagus collapses around ablation cap 82, thus bringing the tissue to be treated in intimate contact with electrodes 28 and viewing window 29. This facilitates uniform electrode contact for even ablation, and improves endoscopic visualization through the viewing window of tissue being treated during the procedure.

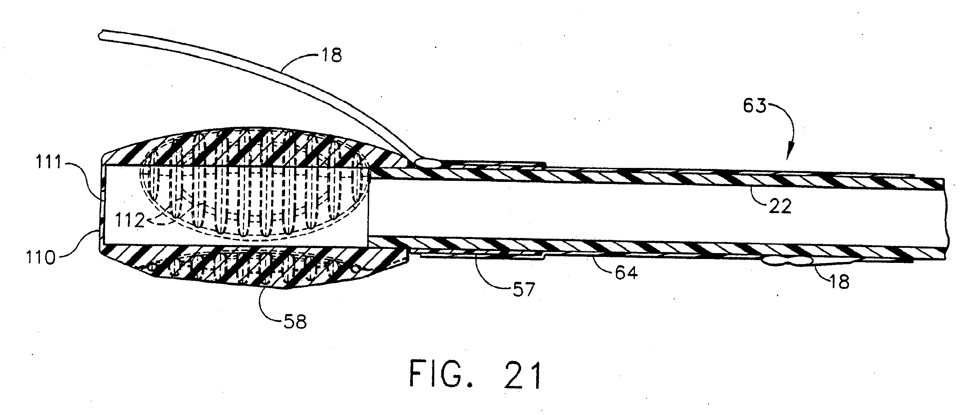

Figure 21 is a sectional view of the proximal portion of sheath 63, rotation knob 58, and conductors 18 of the endoscopic ablation system 10 shown in Figure 17. Rotation knob 58 is molded from a flexible material such as a biocompatible rubber. The proximal end of rotation knob 58 includes a proximal seal 110 having a hole 111 for insertion of endoscope 12 (not shown). The interior of the sheath distal to proximal seal 110 and the interior of ablation cap 82 define an enclosure that is in fluid communication with the interior of the esophagus and the aspiration means of the flexible endoscope 12. Proximal seal 110 prevents fluid communication between the air external to the patient and the interior of sheath 63 and the interior of ablation cap 82. This allows the technique described for Figures 18, 19, and 20 for using the suction available with endoscope 12 to pull the interior of the esophagus into intimate contact with electrodes 28 and viewing window 29. Seal 110 also wipes bodily fluids from the exterior of endoscope 12 as it is withdrawn from sheath 63. Rotation knob 58 also includes a distal cylindrical extension 57 that fits tightly over the proximal end of a rotation tube 22 of sheath 63. An external tube 64 fits tightly over the entire length of sheath 63, including the portion attached to distal cylindrical extension 57 of rotation knob 58. Rotation tube 22 may be made of any one of a number of flexible tubing materials, including corrugated polyethylene tubing. External tube 64 is preferably made from polyolefin that is shrink-wrapped tightly onto rotation tube 22 by the application of heat during assembly. In Figure 21, conductors 18 are shown wrapped around the outside of sheath 63. Conductors 18 may also be assembled between rotation tube 22 and external tube 64 so that the outside of sheath 63 is relatively smooth for passage into the esophagus. Rotation knob 58 also includes a plurality of grip projections to facilitate manipulation.

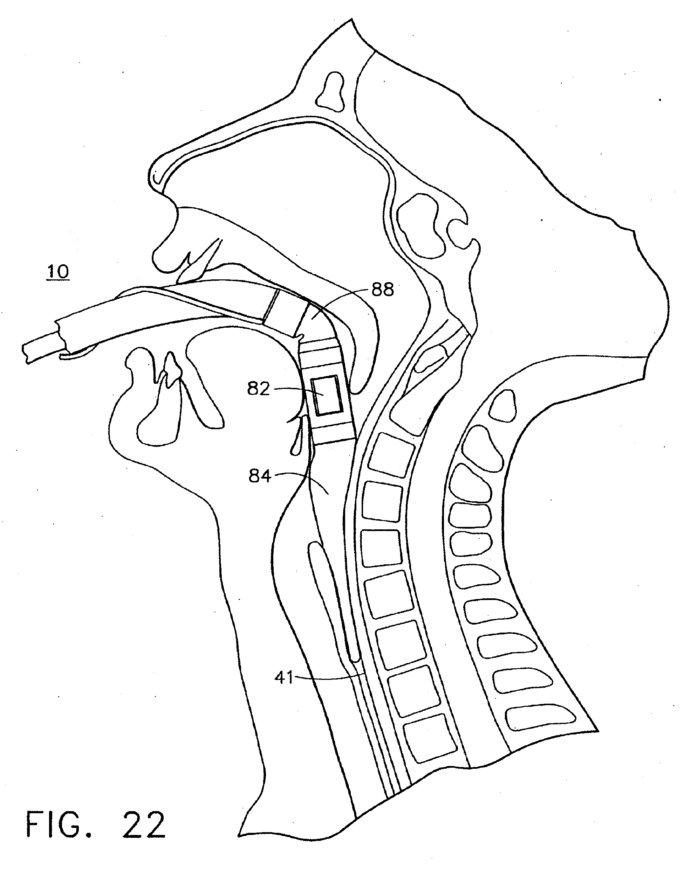

Figure 22 shows the distal portion of endoscopic ablation system 10 of Figure 17 partially inserted into the esophagus 41 of a patient. Tapered end cover 84 dilates esophagus 41 as the operator gently inserts ablation cap 82 for positioning near tissue to be ablated. Flexible coupling 88 flexes as shown, reducing the required insertion force and minimizing trauma (and post-procedural pain) to the patient.

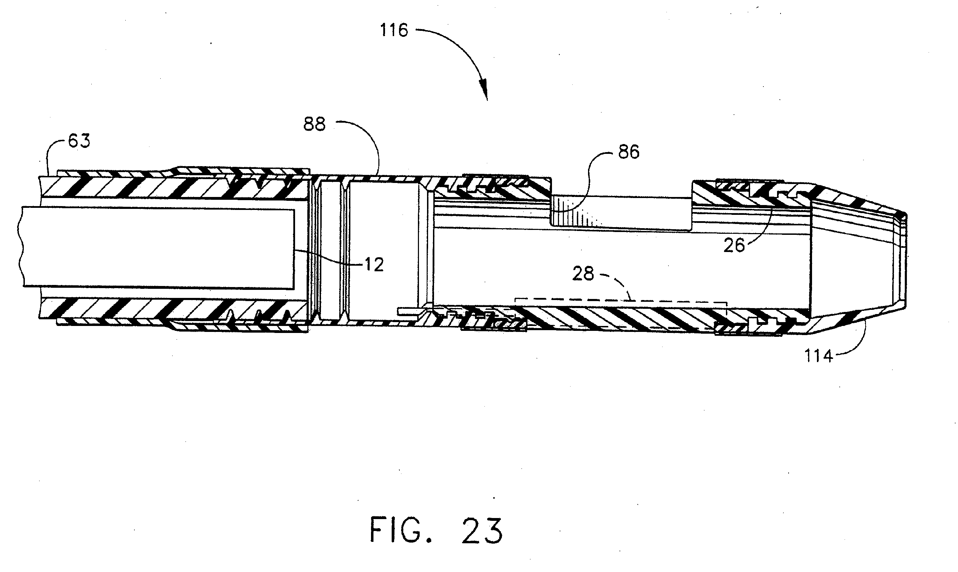

Figure 23 is a sectional view of the distal portion of a further embodiment of an endoscopic ablation system 10. Figure 23 shows an endoscope 12 inserted into an ablation cap 116 that includes a sheath 63, a plurality of electrodes 28, and a flexible coupling 88 such as was described for Figure 19. However the embodiment in Figure 23 includes an open-end piece 114 (also referred to as a tapered end cover) attached to the distal end of rigid support member 26. Open-end piece 114 resembles tapered end cover 84 of Figure 17, but with all but the proximal portion cut off perpendicular to the longitudinal axis. The remaining taper of open-end piece 114 facilitates passage through the esophagus and substantially prevents body fluids on the esophageal wall from collecting inside ablation cap 116. Open-end piece 114 is made preferably from a flexible material such as silicone rubber. The operator may extend the distal end of endoscope 12 through open-end piece 114, to facilitate endoscopic visualization during intubation of ablation cap 116 into the esophagus. The operator may retract endoscope 12 to a retracted position as shown in Figure 23 in order to view tissue through a viewing window (not shown) between adjacent electrodes 28, and to watch the progress of ablation.

Now referring again to Figure 3, the size, shape, and relative position of electrodes 28 are shown, as they would be mounted on rigid support member 26. The region between electrodes 28 forms the viewing window 29. In an endoscopic ablation system according to the present invention, the size, shape and relative position of electrodes 28 are established by the Ablation Index, I, and:

$$I = P/d \qquad (1)$$

Where:

P is the perimeter of electrodes 28 and
d is the separation between adjacent edges 8 of electrodes 28.

In the embodiment of the invention illustrated in Figure 3:

$$I = 2(w+L)/d \qquad (2)$$

Where:

w is the width of electrodes 28 and
L is the length of electrodes 28.

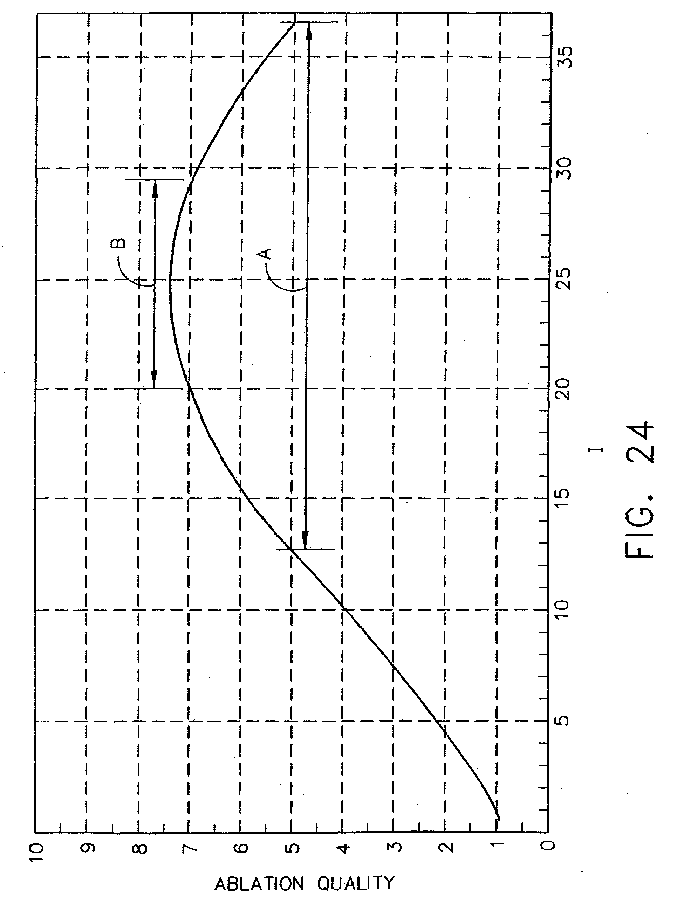

Although the electrodes illustrated in Figure 3 are rectangular in shape, other shapes having an Ablation Index I according to Equation 1 are appropriate for use in the present invention provided that d is substantially constant, i.e. the adjacent edges of the electrodes are substantially parallel. In an endoscopic ablation system according to the present invention, I can be between about 1 and about 200, more particularly between about 15 and 35, such as indicated by a region "A" in the graph of Figure 24. The graph of Figure 24 was based on data derived from experiments with many different electrode geometries for many different conditions. Ablation Quality is a subjective rating of between 1-10 based primarily on area, depth, and color of ablation achieved. Region A indicates the Ablation Index I for when Ablation Quality is greater than or equal to 5 (an average subjective rating) on a scale of 1-10. In some cases, the operator may desire to maintain an ablation index where $20<I<28$, as indicated by a region "B" in Figure 24. Practical considerations related to manufacture, type of tissue being treated, physician preferences, and so on, come into play when determining electrode geometry and selecting an ablation index range. The Ablation Index is used to define an electrode arrangement that substantially confines the initial ablation to the tissue under the viewing window, allowing the surgeon to control the ablation process. In operation, an endoscopic ablation device according to the present invention includes electrodes having an Ablation Index within the prescribed ranges. Such an endoscopic ablation instrument will begin to ablate tissue when an electric potential is established between the electrodes (i.e. the electrodes are actuated). However, during the initial ablation process little or none of the tissue directly beneath the electrodes will be ablated and the thermal profile within the treated tissue will have a substantially vertical wall at the edge of the electrodes. Further, the current density of the electrical current flowing between the electrodes will be very high in the tissue under the viewing window, accelerating the ablation of tissue within the treatment region, giving the surgeon precise control of the treatment region and limiting the ablation of healthy tissue. The operator further has precise control of the degree to which the treated tissue is ablated since the operator may view the entire treatment region through the viewing window. The operator may visually determine when the treated tissue is sufficiently ablated by watching to see when the ablated tissue fills the entire ablation window. When the ablated tissue fills the entire ablation window, the mucosa is consistently ablated to a predetermined depth across the treatment region. The actual depth of the ablation is a function of a number of variables, including power. Uniform ablation depths of approximately one to two millimeters are constantly obtainable using the color of the treated tissue in the ablation window as a guide. Ablation depths of one to two millimeters are normally enough to ablate the abnormal tissue in the mucosa without significantly damaging the healthy tissue underneath.

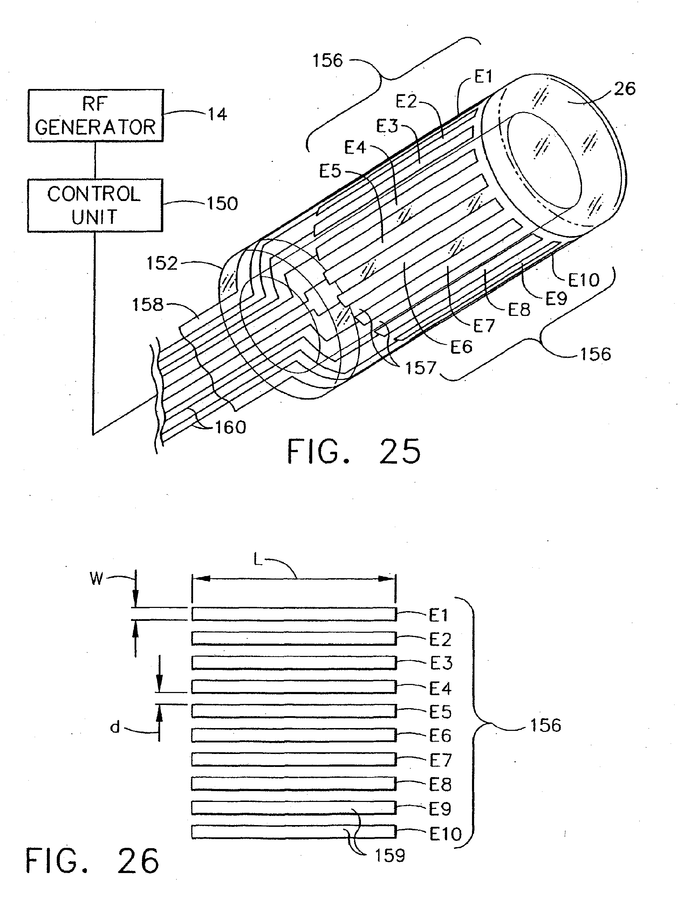

Figure 25 represents an endoscopic ablation system 9 comprising an ablation cap 152, a control unit 150, and a RF generator 14. Ablation cap 152 includes a plurality of electrodes 156, each of which is electrically connected to control unit 150. In this embodiment, ten electrodes labeled E1 through E10 comprise plurality of electrodes 156, and are printed using conventional printed circuit manufacturing techniques onto a transparent substrate 158 made from a material such as clear polyacetate or Mylar film. Transparent substrate 158 is adhered to a rigid support member 154 using, for example, UV cured optical adhesive No. NOA 68, which is available from Norland Products, Inc., New Brunswick, NJ. A plurality of electrode leads 160 are also printed onto substrate 158 and terminate at a solder pad (not shown) for electrical attachment to insulated wires (not shown) for electrical connection to control unit 150. Rigid support member 154 may be identical to rigid support member 26 shown in Figure 2. The proximal end of ablation cap 152 attaches to flexible shaft 32 (see Figure 1). Electrode leads 160 and portions of electrodes 156 may be covered with a dielectric coating or shrink wrap film in order to be insulated from tissue. In this embodiment, a separate electrode lead is provided for each electrode so that each electrode may be individually actuated by control unit 150 according to a predetermined sequence and for a predetermined duration. This enables a large number of different combinations of electrode actuation sequences and durations to obtain desired tissue ablation effects. It is also possible to have more than one electrode attached to a common lead. Because rigid housing member 154 is made of a clear material such as polycarbonate, a plurality of viewing windows are provided in the spaces between electrodes 156 for endoscopically viewing tissue during the ablation procedure.

Figure 26 shows plurality of electrodes 156 of Figure 25 as they would appear laid flat. In this embodiment, each of electrodes E1 through E10 has a rectangular shape with length "L" and width "w", and the distance between the parallel edges of adjacent electrodes is "d". As described for Figure 3, an Ablation Index, I, establishes the size, shape and relative position of electrodes 156 according to the following:

$$I = P/d \qquad = 2(w+L)/d \qquad\qquad (3)$$

Where:

P is the perimeter of electrodes 156

Although the electrodes illustrated in Figure 26 are rectangular in shape, other shapes having an Ablation Index, I, according to Equation 3 are appropriate for use in the present invention provided that d is substantially constant. That is, the adjacent edges of the electrodes should be a constant distance apart along the length of the adjacent electrodes. Therefore, it is possible for electrodes 156 to have a curvilinear shape. As described earlier, I can be between about 1 and about 200, more particularly between about 15 and about 35 such as indicated by a region "A" in the graph of Figure 24. In addition, all of electrodes 156 do not necessarily need to have the same width, length, or distance between electrodes 156. In other embodiments, for example, Ablation Index may vary between pairs of adjacent electrodes to obtain desired tissue ablation effects.

Again referring to Figure 25, control unit 25 comprises generally an internal switching network for activating plurality of electrodes 156 according to a predetermined sequence and pattern. When any two adjacent electrodes 156 have opposite polarities and are in intimate contact with tissue, the tissue between those two adjacent electrodes is ablated, and tissue underneath the two adjacent electrodes 156 is not ablated. Control unit 25 comprises a programmable, multiplexing system for actuating electrodes 156 and is easily constructable by those skilled in the art. Examples of predetermined sequences of actuation are shown in the following tables where E1-E10 refer to electrodes; T1-T9 refer to time periods, (+) indicates positive polarity, (-) indicates negative polarity, and a blank indicates electrode not energized during the specified time period:

Table 1

|    | E1 | E2 | E3 | E4 | E5 | E6 | E7 | E8 | E9 | E10 |
|----|----|----|----|----|----|----|----|----|----|-----|
| T1 | +  | -  |    |    |    |    |    |    |    |     |
| T2 |    | +  | -  |    |    |    |    |    |    |     |
| T3 |    |    | +  | -  |    |    |    |    |    |     |
| T4 |    |    |    | +  | -  |    |    |    |    |     |
| T5 |    |    |    |    | +  | -  |    |    |    |     |
| T6 |    |    |    |    |    | +  | -  |    |    |     |
| T7 |    |    |    |    |    |    | +  | -  |    |     |
| T8 |    |    |    |    |    |    |    | +  | -  |     |
| T9 |    |    |    |    |    |    |    |    | +  | -   |

Table 2

|    | E1 | E2 | E3 | E4 | E5 | E6 | E7 | E8 | E9 | E10 |
|----|----|----|----|----|----|----|----|----|----|-----|
| T1 | -  | +  | -  |    |    |    |    |    |    |     |
| T2 |    | -  | +  | -  |    |    |    |    |    |     |
| T3 |    |    | -  | +  | -  |    |    |    |    |     |
| T4 |    |    |    | -  | +  | -  |    |    |    |     |
| T5 |    |    |    |    | -  | +  | -  |    |    |     |
| T6 |    |    |    |    |    | -  | +  | -  |    |     |
| T7 |    |    |    |    |    |    | -  | +  | -  |     |
| T8 |    |    |    |    |    |    |    | -  | +  | -   |
| T9 |    |    |    |    |    |    |    |    | -  | +   |

Table 3

|    | E1 | E2 | E3 | E4 | E5 | E6 | E7 | E8 | E9 | E10 |
|----|----|----|----|----|----|----|----|----|----|-----|
| T1 | -  | +  | -  |    |    |    |    |    |    |     |
| T2 |    |    | -  | +  | -  |    |    |    |    |     |
| T3 |    |    |    |    | -  | +  | -  |    |    |     |
| T4 |    |    |    |    |    |    | -  | +  | -  |     |
| T5 |    |    |    |    |    |    |    |    | -  | +   |

Table 4

|    | E1 | E2 | E3 | E4 | E5 | E6 | E7 | E8 | E9 | E10 |
|----|----|----|----|----|----|----|----|----|----|-----|
| T1 | -  | +  | -  | +  | -  | +  | -  | +  | -  | +   |
|    |    |    |    |    |    |    |    |    |    |     |

In Table 1, electrodes E1 and E2 are energized (on) at time T1, while electrodes E3 through E10 are not energized (off). At time T2, electrodes E2 and E3 are on, while electrodes E1 and E4 through E10 are off, and so on until all tissue in the viewing windows is ablated. The duration of each actuation may vary, but can be approximately 1-2 seconds in one embodiment. By energizing electrodes 156 sequentially in this manner, the peak power requirement for RF generator 14 is significantly less than if all the electrodes 156 were energized simultaneously. Also, while all the electrodes could be energized simultaneously as in Table 4, it may be desirable to energize the electrodes in a sequential manner, as in Tables 1 and 2, so that the tissue ablation can be observed as it occurs through the appropriate window.

A physician may use endoscopic ablation system 9 shown in Figure 25 in the same manner as was described for endoscopic ablation system 10 of Figures 1-5, with one primary difference. That is, the physician will not need to rotate endoscopic ablation system 9 as often within the body lumen as would be required for endoscopic ablation system 10, due to the larger number of electrodes 156 on the former. If the electrodes 156 are disposed around substantially the entire perimeter of the ablation cap, then the device could be rotated within the body lumen only once in either direction, and approximately by a distance equal to the width, w, of an electrode 156, to provide ablation of the tissue around the circumference of the lumen.

In Tables 2 and 3, three electrodes are energized simultaneously. Ten electrodes are shown in Figure 26, but it will be understood that more electrodes or fewer electrodes could be used, as desired.

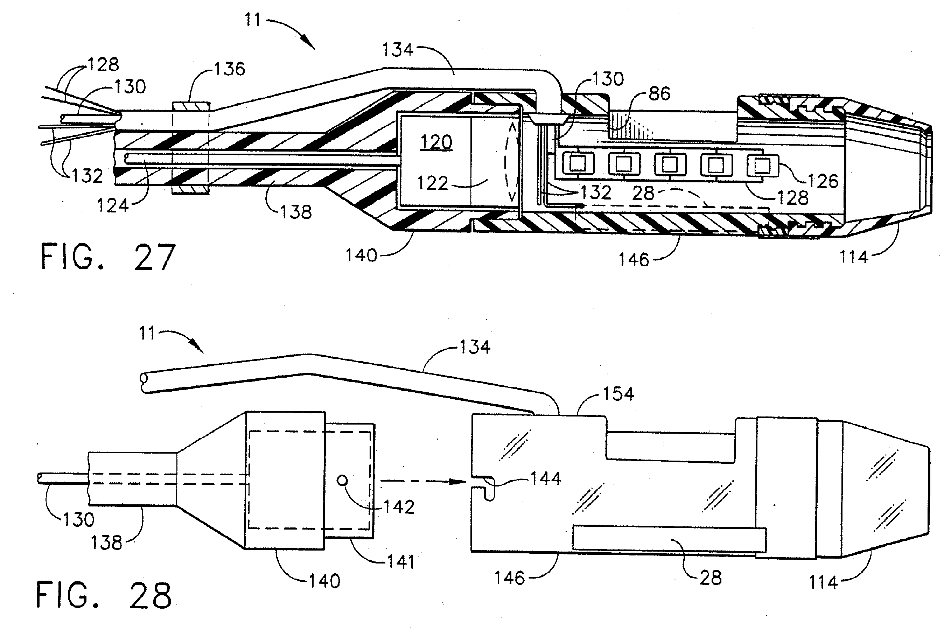

Figure 27 is a sectional view of the distal portion of an endoscopic ablation system 11 including a distally mounted image sensor 120. A flexible endoscope and a conventional video tower are not required for visualization of tissue. Endoscopic ablation system 11 comprises a flexible shaft 138 and a detachable ablation cap 146. Endoscopic ablation system 11 can also be constructed so that ablation cap 146 is not detachable from flexible shaft 138. Flexible shaft 138 includes a sensor housing 140 that contains image sensor 120. Image sensor 120 may be a CMOS (Complementary Metallic Oxide Sensor) camera such as Model Number OV7910, which is available from Omnivision Technologies, Inc. (www.ovt.com). Image sensor 120 may include an objective lens 122 as shown in this embodiment, or may be a pin-hole style CMOS camera that may be used with red light LED illumination, for example. A CMOS cable 124 passing through flexible shaft 138 contains a signal wire for connection to a NTSC or PAL formatted display monitor and a pair of electrical leads for connection to a 5VDC-power supply.

Still referring to Figure 27, ablation cap 146 comprises a rigid support member 154 made of a clear plastic such as polycarbonate, and may have approximately the same configuration as rigid support member 26 described for Figure 18. Rigid support element 154 is hollow and has an inner surface 162 and an outer surface 164. A plurality of illuminators 126 are surface mounted on inner surface 162 in order to illuminate the field of view of image sensor 120. White light, surface mounted LED's such as Model No. NSPWF50BS available from Nichia (www.nichia.co.jp) are suitable as illuminators 126. Illuminator leads 128 electrically connect in parallel illuminators 126 to a DC power supply (not shown). An umbilical tube 134 has a distal end attached to rigid support member 154 and is long enough to extend outside of the body lumen. Umbilical tube 134 removably attaches to flexible shaft 138 with at least one clip 136. Umbilical tube 134 contains illuminator leads 128, a plurality of bipolar electrode leads 132, and a suction tube 130, which is connected to a vacuum source (not shown).

Figure 28 is a side view of the distal portion of endoscopic ablation system 11 shown in Figure 27. In Figure 28, ablation cap 146 and umbilical tube 134 are shown detached from flexible shaft 138, thus allowing cleaning and reuse of a hermetically sealed and cleanable version of flexible shaft 138 containing image sensor 120. Ablation cap 146 and umbilical tube 134 transport body fluids and support components, especially electrodes 128, that may degrade with repeated use, and therefore may be fabricated as single patient use, disposable components. Figure 28 shows one of many variations of attaching ablation cap 146 to flexible shaft 138. Each of at least one retaining slots 144 engages with a corresponding post 142 projecting radially from a boss 141 on the distal end of flexible shaft 138. (This variation of attaching two components is commonly referred to as a "bayonet fitting.")

Electrodes having an ablation index and viewing window according to the present invention may be used in other surgical instruments such as, for example, endocutters. Further, electrodes having an ablation index according to the present invention may be used for other treatment regimens such as tissue welding, electrophoresis and coagulation of varicose veins and hemorrhoids.

While preferred embodiments of the present invention have been shown and described herein, it will be obvious to those skilled in the art that such embodiments are provided by way of example only. Numerous variations, changes, and substitutions will now occur to those skilled in the art without departing from the invention. Accordingly, it is intended that only the spirit and scope of the appended claims limit the invention.

**Claims**

**1.** An ablation system for electrosurgically treating bodily tissue of a patient, said ablation system comprising:

a plurality of electrodes, each of said electrodes having a perimeter P, wherein adjacent electrodes have adjacent parallel edges spaced apart by a distance d, wherein an ablation index I = P/d is between about 1 and 200;

a plurality of viewing windows, wherein each viewing window is positioned between an adjacent pair of said plurality of electrodes.

2. The ablation system of Claim 1 further comprising an electric current supply for providing current to the plurality of electrodes according to a predetermined sequence.

3. The ablation system of Claim 2 further comprising an electric current supply for providing current to the plurality of electrodes according to a predetermined duration.

4. An ablation system according to Claim 1, wherein said ablation index I is between about 15 and 35.

5. An ablation system according to Claim 1, wherein each of said plurality of electrodes has a rectangular shape with a width w and a length L, wherein P = 2(w+L).

6. An ablation system according to Claim 1, wherein said ablation system further comprises a sheath for receiving an endoscope, and an ablation cap disposed at a distal end of the sheath, wherein the plurality of electrodes are positioned on the ablation cap, and wherein the plurality of viewing windows form a portion of the ablation cap.

7. An endoscopic ablation system according to Claim 1, comprising a hollow ablation end cap mounted on the distal end of a sheath, wherein a distal end of a flexible endoscope may be inserted through said sheath and at least partially into said ablation end cap, and said sheath and said ablation end cap are rotatable with respect to said flexible endoscope.

8. An endoscopic ablation system according to Claim 1 comprising an ablation cap having a tapered end cover.

9. An endoscopic ablation system according to Claim 8, wherein the tapered end cover has a closed configuration, and wherein the tapered end cover is adapted to open in order to allow passage of the distal end of an endoscope therethrough.

10. An endoscopic ablation system according to Claim 8, wherein said tapered end cover is normally open and is adapted to allow passage of the distal end of an endoscope therethrough.

FIG. 1

RF GENERATOR 14

EP 1 402 837 A1

FIG. 2

FIG. 3

FIG. 4

FIG. 5

FIG. 6

FIG. 7

FIG. 8

FIG. 9

FIG. 10

EP 1 402 837 A1

FIG. 11

EP 1 402 837 A1

FIG. 12

FIG. 13

EP 1 402 837 A1

FIG. 14

FIG. 15

FIG. 16

FIG. 17

FIG. 18

FIG. 19

FIG. 20

EP 1 402 837 A1

FIG. 21

EP 1 402 837 A1

FIG. 22

FIG. 23

EP 1 402 837 A1

FIG. 24

RF GENERATOR — 14

CONTROL UNIT — 150

156

E1
E2
E3
E4
E5

26

152

158

E10
E9
E8
E7
E6

157

156

160

FIG. 25

W

L

E1
E2
E3
E4
E5
E6
E7
E8
E9
E10

d

156

159

FIG. 26

FIG. 27

FIG. 28

EP 1 402 837 A1

**European Patent Office**

## EUROPEAN SEARCH REPORT

Application Number

EP 03 25 5838

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 99 00060 A (ADVANCED CORONARY INTERVENTION) 7 January 1999 (1999-01-07) | 1-10 | A61B18/14 |
| X | * page 23, line 11 - page 24, line 2; claims 23-25; figure 7 * | 1 | |
| X | * the whole document * | 2-10 | |
| | --- | | |
| Y | WO 01 05318 A (CURON MEDICAL INC ;GAISER JOHN (US); UTLEY DAVID (US); CROFT RACHE) 25 January 2001 (2001-01-25) * page 27, column 5 - column 18; figures 1-29 * | 1-10 | |
| | --- | | |
| Y | WO 01 24721 A (CURON MEDICAL INC) 12 April 2001 (2001-04-12) * the whole document * | 1-10 | |
| | --- | | |
| A | WO 01 35846 A (ZELICKSON BRIAN D ;GANZ ROBERT A (US); STERN ROGER A (US)) 25 May 2001 (2001-05-25) * the whole document * | 1-10 | |
| | --- | | TECHNICAL FIELDS SEARCHED (Int.Cl.7) |
| A | US 6 022 334 A (LUNDQUIST INGEMAR H ET AL) 8 February 2000 (2000-02-08) * the whole document * | 1-10 | A61B |
| | --- | | |
| A | WO 02 47569 A (BROWN CHARLES E III ;MACADAM DAVID (US); BARD INC C R (US); SAGON) 20 June 2002 (2002-06-20) * the whole document * | 1-10 | |
| | ----- | | |

The present search report has been drawn up for all claims

| Place of search | Date of completion of the search | Examiner |
|---|---|---|
| MUNICH | 18 December 2003 | BIRKENMAIER, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                 
& : member of the same patent family, corresponding document

EPO FORM 1503 03.82 (P04C01)

ANNEX TO THE EUROPEAN SEARCH REPORT
ON EUROPEAN PATENT APPLICATION NO.

EP 03 25 5838

This annex lists the patent family members relating to the patent documents cited in the above-mentioned European search report.
The members are as contained in the European Patent Office EDP file on
The European Patent Office is in no way liable for these particulars which are merely given for the purpose of information.

18-12-2003

| Patent document cited in search report | | Publication date | Patent family member(s) | | Publication date |
|---|---|---|---|---|---|
| WO 9900060 | A | 07-01-1999 | WO | 9900060 A1 | 07-01-1999 |
| WO 0105318 | A | 25-01-2001 | US | 6645201 B1 | 11-11-2003 |
| | | | AU | 6087500 A | 05-02-2001 |
| | | | CA | 2378067 A1 | 25-01-2001 |
| | | | EP | 1196106 A1 | 17-04-2002 |
| | | | JP | 2003520069 T | 02-07-2003 |
| | | | WO | 0105318 A1 | 25-01-2001 |
| | | | US | 2002115992 A1 | 22-08-2002 |
| WO 0124721 | A | 12-04-2001 | US | 6405732 B1 | 18-06-2002 |
| | | | AU | 7724200 A | 10-05-2001 |
| | | | CA | 2385689 A1 | 12-04-2001 |
| | | | EP | 1223881 A1 | 24-07-2002 |
| | | | JP | 2003510160 T | 18-03-2003 |
| | | | WO | 0124721 A1 | 12-04-2001 |
| | | | US | 2002138075 A1 | 26-09-2002 |
| WO 0135846 | A | 25-05-2001 | AU | 1617401 A | 30-05-2001 |
| | | | CA | 2388861 A1 | 25-05-2001 |
| | | | EP | 1229849 A1 | 14-08-2002 |
| | | | WO | 0135846 A1 | 25-05-2001 |
| | | | US | 2003158550 A1 | 21-08-2003 |
| | | | US | 6551310 B1 | 22-04-2003 |
| US 6022334 | A | 08-02-2000 | US | 5749846 A | 12-05-1998 |
| | | | US | 5435805 A | 25-07-1995 |
| | | | US | 5366490 A | 22-11-1994 |
| | | | US | 5370675 A | 06-12-1994 |
| | | | AT | 191328 T | 15-04-2000 |
| | | | AU | 685086 B2 | 15-01-1998 |
| | | | AU | 6133194 A | 29-08-1994 |
| | | | AU | 718834 B2 | 20-04-2000 |
| | | | AU | 6189698 A | 09-07-1998 |
| | | | AU | 6823494 A | 12-12-1994 |
| | | | CA | 2155217 A1 | 18-08-1994 |
| | | | DE | 4416902 A1 | 24-11-1994 |
| | | | DE | 69423826 D1 | 11-05-2000 |
| | | | DE | 69423826 T2 | 12-10-2000 |
| | | | DK | 637436 T3 | 04-09-2000 |
| | | | EP | 0637436 A1 | 08-02-1995 |
| | | | EP | 0631514 A1 | 04-01-1995 |
| | | | ES | 2146637 T3 | 16-08-2000 |
| | | | FR | 2705242 A1 | 25-11-1994 |
| | | | GR | 3033763 T3 | 31-10-2000 |
| | | | IL | 108532 A | 13-07-1997 |

EPO FORM P0459

For more details about this annex : see Official Journal of the European Patent Office, No. 12/82

35

# EP 1 402 837 A1

**ANNEX TO THE EUROPEAN SEARCH REPORT
ON EUROPEAN PATENT APPLICATION NO.**

EP 03 25 5838

This annex lists the patent family members relating to the patent documents cited in the above-mentioned European search report.
The members are as contained in the European Patent Office EDP file on
The European Patent Office is in no way liable for these particulars which are merely given for the purpose of information.

18-12-2003

| Patent document cited in search report | | Publication date | Patent family member(s) | | Publication date |
|---|---|---|---|---|---|
| US 6022334 | A | | IL | 109544 A | 10-06-1997 |
| | | | JP | 8506259 T | 09-07-1996 |
| | | | PT | 637436 T | 31-08-2000 |
| | | | SI | 637436 T1 | 31-12-2000 |
| | | | US | 6102886 A | 15-08-2000 |
| | | | WO | 9417856 A1 | 18-08-1994 |
| | | | WO | 9426187 A1 | 24-11-1994 |
| | | | US | 6241702 B1 | 05-06-2001 |
| | | | US | 5409453 A | 25-04-1995 |
| | | | US | 5486161 A | 23-01-1996 |
| | | | US | 5470308 A | 28-11-1995 |
| | | | US | 5556377 A | 17-09-1996 |
| | | | US | 5720718 A | 24-02-1998 |
| | | | US | 5542915 A | 06-08-1996 |
| | | | US | 5470309 A | 28-11-1995 |
| | | | US | 5554110 A | 10-09-1996 |
| | | | US | 5549644 A | 27-08-1996 |
| | | | US | 5456662 A | 10-10-1995 |
| | | | US | 5630794 A | 20-05-1997 |
| | | | US | 5514131 A | 07-05-1996 |
| | | | US | 5720719 A | 24-02-1998 |
| | | | US | 5667488 A | 16-09-1997 |
| | | | US | 5531677 A | 02-07-1996 |
| | | | US | 5685839 A | 11-11-1997 |
| | | | US | 5599295 A | 04-02-1997 |
| | | | US | 5607389 A | 04-03-1997 |
| | | | US | 5741225 A | 21-04-1998 |
| | | | US | 5681277 A | 28-10-1997 |
| | | | US | 5718702 A | 17-02-1998 |
| WO 0247569 | A | 20-06-2002 | EP | 1343426 A1 | 17-09-2003 |
| | | | EP | 1343427 A1 | 17-09-2003 |
| | | | WO | 02056783 A1 | 25-07-2002 |
| | | | WO | 0247569 A1 | 20-06-2002 |

EPO FORM P0459

For more details about this annex : see Official Journal of the European Patent Office, No. 12/82

36